# EUROPEAN PATENT APPLICATION

(11) **EP 2 206 530 A1**
(43) Date of publication of application: **14.07.2010**
(21) Application number: 09150137.9
(22) Date of filing: 07.01.2009
(51) Int. Cl.: A61M 37/00

(54) **Pigment supply system for a pigment dispenser**

(71) Applicant: Freedom Tattoo, 1501 CN Zaandam (NL)
(72) Inventor: Loach, Scott Edward, 1501 AK, Zaandam (NL)
(74) Representative: Metman, Karel Johannes

(57) **Abstract**

A pigment supply system for a pigment dispenser (2), such as a tattoo pen, comprising a disposable pigment reservoir (8; 18), and an adapter (7) including a hollow member (10) for insertion into the reservoir and having pigment supply means (13) to control the supply of pigment from the reservoir through the hollow member to the pigment dispenser. The adapter may be constructed as a clip to be clipped on a tip (6') of the pigment dispenser (2), or may be integrated in the tip (6"). The reservoir may be a standard ink cartridge for ink pens filed with pigment, or may be formed from standard pigment cap (16) and a coupling device (17).

## Description

The invention relates to an pigment supply system for a pigment dispenser, such as a tattoo pen.

In the present practice tattoo pens are supplied with pigment from large pigment (ink) containers. For each tattoo several doses of pigment must be transferred from the container to the tattoo pen which is of course very inconvenient. Furthermore, the sterility of the pigment in the pigment containers can not be guaranteed, even when the tattoo artist works under very hygienic conditions.

Various attempts have been made to improve the pigment supply system for tattoo pens and the like, see for example US 4,671,277, DE 299 16 971 U1 and US 6,505,530 B2. Despite the attempts, none of these systems have reached practice.

It is an object of the present invention to provide a novel pigment supply system that is easy to use and can be made in a cost effective way.

For this purpose, the invention provides a pigment supply system for a pigment dispenser, such as a tattoo pen, comprising a disposable pigment reservoir, and an adapter including a hollow member for insertion into the reservoir and having pigment supply means to control the supply of pigment from the reservoir through the pin to the pigment dispenser.

Due to the (sterile) disposable pigment reservoir that can be connected to the adapter of the pigment dispenser, the risk of spreading contamination is greatly reduced and it confines any pigment/bodily fluid contaminants to the specific work area of the pigment dispenser. Furthermore, the system is much more convenient to use as the reservoir will normally contain sufficient pigment for a normal tattoo. If more pigment is needed for a tattoo, it is only necessary to exchange the empty reservoir for a new sterilized one.

It is most convenient that the reservoir is a sterile standard ink pen cartridge filled with pigment and having a seal, such as a ball closing off the supply opening, the hollow member being adapted to break the seal to open the reservoir.

By using such widely available pigment reservoirs, the cost can be kept to a minimum. Of course, it is possible to provide for reservoirs having various volumes in order to allow a selection of the reservoir based on the supply of pigment needed for a tattoo or the like.

Preferably, the supply means include plastic bristles being fastened in the hollow member and protruding therefrom to such an extent that they are able to come into contact with a needle bar in the pigment dispenser.

Due to this very simple supply means, pigment is only supplied from the reservoir to the pigment dispenser when the needle bar is oscillating so that the operation of the tattoo machine automatically starts and stops the supply of the pigment to the pigment dispenser. The principle of the supply means is based on the fact that the bristles will normally close off the pigment reservoir, but pigment will be supplied automatically when the bristles are vibrated as a result of the oscillating motion of the needle bar.

The adapter including the hollow member and the pigment supply means may be constructed as a clip which is adapted to be clipped onto a tip of a tattoo machine, preferably by clipping it over a lateral opening thereof.

By means of this clip, it is possible to attach the reservoir to a reusable or disposable standard tube/grip/tip assembly used nowadays, so that they can remain in service with the added supply system which facilitates the introduction of the new pigment supply system. On the other hand, it is conceivable to integrate the adapter in the tip of the disposable pigment dispenser, so that they form a unit to which the pigment reservoir can be connected, which makes it even easier to assemble the various parts of the pigment dispenser.

The invention also includes a pigment dispenser, a pigment reservoir and adapter as described above.

Further details and advantages of the invention will become clear from the following description of embodiments of the invention with reference to the accompanying drawings, in which:
Figure 1 is a schematic side view of a tattoo machine with attached disposable tube/grip/tip needle assembly and pigment supply system in operational position in the hand of a user,
Figure 2 is a perspective view of the pigment supply system of Figure 1, on a larger scale,
Figure 3 is an sectional view along the line III-III in Figure 2, wherein the needle bar has been shown partially for illustrating purposes,
Figure 4 is an exploded sectional view of the pigment supply means of Figure 3 to illustrate the assembly of parts,
Figure 5 is a side view of the pigment dispenser tip with added pigment supply system to illustrate the addition thereof,
Figure 6 is a side view of the tube/grip/tip needle assembly of Figure 1, with replacement tip with integrated pigment supply system,
Figure 7 is a side view of a second embodiment of a reservoir for the pigment supply system according to the invention, and
Figure 8 is an exploded view of the parts of the pigment reservoir of Figure 7.

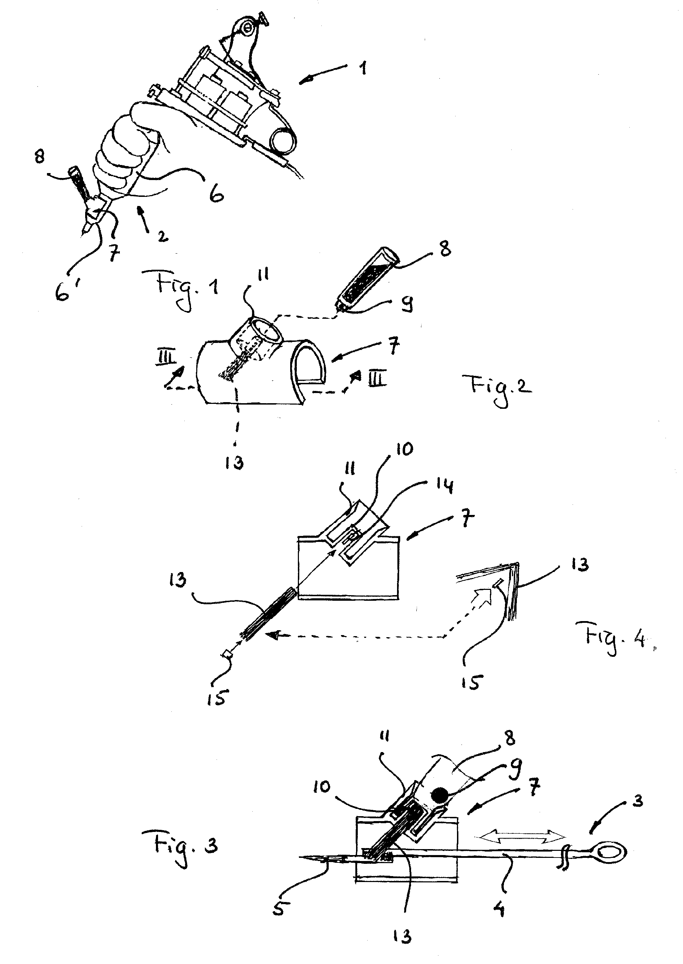

Figure 1 shows a tattoo machine 1, with a pigment dispenser 2 in the form of a tube/grip/tip and needle assembly attached to it. The tattoo machine includes a drive mechanism (not shown) for a needle bar assembly 3. This disposable needle bar assembly 3 includes a bar 4 having a member, such as an eyelet to be connected to the drive mechanism and a needle 5 attached to the bar 4. The needle tip is adapted to supply pigment into the skin, actually into the dermis, of a human body or the like. In case of a disposable unit at least the tube and grip of the pigment dispenser 2 are formed into an integral housing 6 that can be held by the tattoo artist to move the needle 5 along the skin, while the tip 6' may be formed as a separate part that can be inserted into the grip. Of course, it is possible to use the invention for a reusable tube/grip/tip assembly, made from metal/rubber/metal or the like, as well.

Figure 1 shows that for supplying pigment (ink) to the needle 5 there is provided an adapter 7 which is able to receive a pigment reservoir 8 on the one hand and to connect to the tip 6' of the housing 6 on the other hand. This adapter 7 enables the use of sterile disposable reservoirs 8, which may be provided in the form of a pigment cartridge that is similar to a standard refill cartridge that is used in ink pens, but is now filled with tattoo pigment. Such cartridges are made from soft sterilisable plastic and are substantially cylindrical having an opening on one end which is closed by an openable closure, for example in the form of a ball 9 which is wedged in the opening. This ball 9 seals the opening and this seal can be broken in order to dispose the outlet opening by pushing the ball 9 into the reservoir 8. This can be done by means of a pin-shaped hollow member 10 which is provided in the adapter 7.

As is shown in Figures 2 - 4, the hollow member 10 is provided in the centre of a sleeve 11 adapted to receive the part of the pigment reservoir 8 which extends around the opening thereof. The hollow member 10 can be frictionally mounted in an opening in a transverse wall of the sleeve, or can be integrally formed therewith as is shown. When the adapter 7 is connected to the tip 6' of the pigment dispenser, the centre line of the sleeve 11 is at an angle of ca. 40° - 50° with respect to the needle bar assembly 3 within the housing 6, so that the reservoir 8 is in a substantially upright position when the housing 6 is held in its inclined working position. Of course it is possible to position the sleeve 11 and the coaxial member 10 at a different angle to the tip 6'.

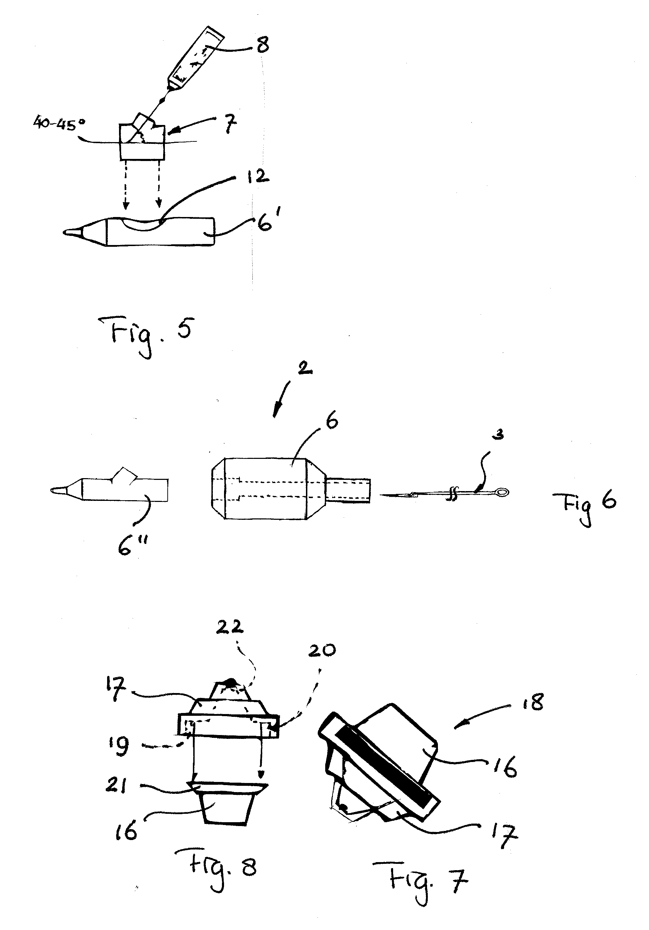

The adapter in the embodiment of Figures 2 - 5 is constructed as a clip that can be clipped onto the tip 6' of the housing 6 so as to close a lateral opening 12 (Fig. 5) in the tip 6'. For this purpose the adapter 7 has a main body that has the form of a slitted cylinder that can elastically deform so that the "legs" on both sides of the sleeve 11 can be pushed around the tip 6' of the housing 6. This cylinder portion of the adapter 7 can be provided with positioning means that engage into the lateral opening 12 to position the adapter 7 with respect to the housing 6. Of course other shapes and configurations are conceivable.

The adapter 7 is furthermore provided with pigment supply means that control the supply of pigment from the pigment reservoir 8 to the needle 5. In this embodiment, the pigment supply means include plastic bristles 13 that are fastened in the hollow member 10 and protrude therefrom to such an extent that in operation they will contact the needle/bar assembly 3. Due to this contact, in operation, the bristles 13 will be vibrated by the needle 5, which oscillates with a frequency of 40 - 80 Hz, and this vibration will cause the pigment to flow from the pigment reservoir 8 along the bristles 13, which are packed against each other in the hollow member 10, to the needle 5 as long as the needle 5 oscillates. In order for the pigment in the reservoir 8 to reach the interior of the hollow member 10, there is provided a slot 14 or other opening in the side of the hollow member 10 which is positioned at least partly in the interior of the pigment reservoir 8 when this is in its operational position on the hollow member 10. The top of the hollow member 10 may be closed and slightly conical to facilitate this top of the hollow member 10 to push against the ball 9 in the pigment reservoir 8 in order to break the seal and open the reservoir. The bristles 13 may be mounted in the hollow member 10 by wedging a bunch of bristles into the hollow member 10, such that each pair of bristles is formed from a larger bristle folded halfway. A small plastic clip 15 wedged into the hollow member 10 may be used to hold the bristles in position.

Figure 6 shows a second embodiment of the pigment supply system, in which the tip 6' is replaced by a tip 6" which has the pigment supply system integrated in it, so that the pigment reservoir can be mounted directly to the tip 6" of the pigment dispenser 2.

Figures 7 and 8 shows a further embodiment of a pigment reservoir 18, which makes use of a standard pigment or ink cap 16 which can be filled by the tattoo artist. This allows the artist to choose pigment personally in respect of colours, water shadow and special mixes. As the tattoo artists are used to the standard pigment caps, the invention provides for a coupling device 17, which is adapted to form a reservoir in combination with the pigment cap 16 on the one hand and to fit onto the adapter 7 on the other hand. For this purpose the coupling device 17 has a circumferential sealing surface 19 to seal the open mouth of the pigment cap 16 and has also means to fasten the pigment cap to the coupling device in a sealed manner, for example by means of ridges 20 that may snap around a circumferential flange 21 around the open mouth of the pigment cap 16. It is also conceivable to use a glue sticker to fix both parts. The coupling device 17 comprises a sealed opening 22, which seal is broken when the coupling device 17 is mated with the hollow member 10 of the adapter 7 in a manner which is comparable to breaking the seal in the pigment reservoir 8. The reservoir 18 can be combined with both embodiments of Figures 1-5 and Figure 6, respectively.

The invention described above and shown in the drawing provides a pigment supply system which is very easy to use and leads to a safe and hygienic pigment supply in tattoo pens and the like.

The invention is not limited to the embodiments shown in the drawing and described above, which can be varied in different ways within the scope of the invention. For example, the pigment supply system may also be used for dispensing permanent make-up. The hollow member in the adapter may take a different shape and the opening may be provided in another position. The pigment supply means may be formed with a slitted rubber bar or other members that can transport pigment in a controlled manner from the reservoir to the needle bar of the pigment dispenser.

## Claims

1. Pigment supply system for a pigment dispenser (2), such as a tattoo pen, comprising a disposable pigment reservoir (8; 18), and an adapter (7) including a hollow member (10) for insertion into the reservoir and having pigment supply means (13) to control the supply of pigment from the reservoir through the hollow member to the pigment dispenser (2).

2. Pigment supply system according to claim 1, wherein the reservoir (8; 18) is a sterile standard ink pen cartridge (8) filled with pigment and having a seal, such as a ball (9) closing off the supply opening, the hollow member (10) being adapted to break the seal to open the reservoir.

3. Pigment supply system according to claim 1 or 2, wherein the supply means (13) includes plastic bristles being fastened in the hollow member (10) and protruding therefrom to such an extent that they are able to come into contact with a needle bar (3) of the pigment dispenser.

4. Pigment supply system according to claim 3, wherein the bristles (13) are fixed in the hollow member (10) by folding them and inserting them into the hollow member by means of a wedge (15) .

5. Pigment supply system according to one of the preceding claims, wherein the top of the pin-shaped hollow member (10) is closed and the side of the hollow member (10) being provided with an opening, such as a slot (14) which is positioned within the reservoir (8; 18) when the hollow member is inserted into the reservoir.

6. Pigment supply system according to one of the preceding claims, wherein the adapter (7) constructed as a clip which is adapted to be clipped onto a disposable tip (6') of a tattoo machine, preferably by clipping it over a lateral opening (12) thereof.

7. Pigment supply system according to one of claims 1 - 5, wherein the adapter (7) is integrated in a disposable tip (6") of a tattoo machine.

8. Pigment supply system according to one of claims 1 or 3-7, wherein the pigment reservoir (18) comprises a standard pigment cap (16) and a coupling device (17) sealing to the pigment cap on one end and to the adapter on the other end.

9. Pigment dispenser comprising a disposable tip (6'; 6"), a needle bar (3) provided therein and adapted to be connected to a tattoo machine (1), and a pigment supply system according to one of the preceding claims.

10. Pigment dispenser according to claim 9, wherein the adapter (7) is integrated in the tip (6").

11. Pigment reservoir for use in the pigment supply system according to one of claims 1 - 8, wherein the pigment reservoir is a standard ink cartridge (8) for ink pens, which is filled with tattoo pigment.

12. Adapter (7) for use in a pigment supply system for a pigment dispenser (2), such as a tattoo pen, comprising a hollow member (10) for insertion into a disposable pigment reservoir (8; 18), and pigment supply means (13) to control the supply of pigment from the reservoir through the hollow member (10) to the pigment dispenser (2).
